# EUROPEAN PATENT SPECIFICATION

(11) **EP 3 138 484 B1**
(45) Date of publication and mention of the grant of the patent: **01.05.2019**
(21) Application number: 16186937.5
(22) Date of filing: 02.09.2016
(51) Int. Cl.: A61B 5/06, A61B 5/042

(54) **IDENTIFYING AND PRESENTING SUSPECTED MAP SHIFTS**
IDENTIFIZIERUNG UND DARSTELLUNG MÖGLICHER KARTENVERSCHIEBUNGEN
IDENTIFICATION ET PRÉSENTATION DE DÉCALAGES DE CARTE SUSPECTÉS

(30) Priority: 04.09.2015 US 201562214262 P; 04.08.2016 US 201615228588
(43) Date of publication of application: 08.03.2017
(73) Proprietor: Biosense Webster (Israel) Ltd., 2066717 Yokneam (IL)
(72) Inventor: LUDWIN, Doron Moshe, 2066717 Yokneam (IL); PERI, Eitan, 2066717 Yokneam (IL); TURGEMAN, Aharon, 2066717 Yokneam (IL); ROSENBERG, Avigdor, 2066717 Yokneam (IL); SCHECHTER, Menachem, 2066717 Yokneam (IL)
(74) Representative: Carpmaels & Ransford LLP

(56) References cited:
- EP-A1- 2 881 037
- EP-A2- 2 674 126
- WO-A2-2009/137558

## Description

### FIELD OF THE INVENTION

The present invention relates generally to medical imaging, and specifically to an apparatus for color-coding ablation locations by their respective registrations.

### BACKGROUND OF THE INVENTION

A wide range of medical procedures involves placing objects, such as sensors, tubes, catheters, dispensing devices, and implants, within the body. Real-time imaging methods are often used to assist doctors in visualizing the object and its surroundings during these procedures. In most situations, however, real-time three-dimensional imaging is not possible or desirable. Instead, systems for obtaining real-time spatial coordinates of the internal object are often utilized.

U.S. Patent Application 2007/0016007, to Govari et al. , describes a hybrid field-based and impedance-based location sensing system. The system includes a probe adapted to be introduced into a body cavity of a subject.

U.S. Pat. No. 6,574,498, to Gilboa, describes a system for determining the position of a work piece within a cavity of an opaque body. The system claims to use a transducer that interacts with a primary field, and several transducers that interact with a secondary field.

U.S. Pat. No. 5,899,860, to Pfeiffer, et al., describes a system for determining the position of a catheter inside the body of a patient. A correction function is determined from the difference between calibration positions derived from received location signals and known, true calibration positions, whereupon catheter positions, derived from received position signals, are corrected in subsequent measurement stages according to the correction function.

European Patent Application Publication EP2881037A1 discloses a method, including acquiring a body cavity image and receiving first and second sets of mapping points from a probe inserted into the cavity. A first registration, having a first cumulative error value, is performed between the first set's mapping points and the image, each of the first set's mapping points having a respective first point error value, and a 3D anatomical map is generated including the first set's mapping points whose respective first point error values are less than a specified threshold. A second registration is performed between the received mapping points and the image, each of the received mapping points having a respective second point error value, the second registration having a second cumulative error value lower than the first cumulative error value, and an updated 3D anatomical map is generated including the received mapping points whose respective second point error values are less than the specified threshold.

The description above is presented as a general overview of related art in this field and should not be construed as an admission that any of the information it contains constitutes prior art against the present patent application.

### SUMMARY OF THE INVENTION

The invention is defined in the appended claims.

There is provided, in accordance with an embodiment of the present invention an apparatus, including a tracking system configured to track a location of a probe within an organ of a human body, a baseline coordinate system, a display, and a processor configured to perform a first registration of the tracking system with the baseline coordinate system, to measure first locations of the probe within the organ following the first registration, to present, on the display, first indicators on the organ marking the first locations with a first visual effect, at positions on the image that are determined in accordance with the first registration, after measuring the first locations, to perform a second registration of the tracking system with the baseline coordinate system, to measure second locations of the probe within the organ following the second registration, and to present, on the image of the organ, second indicators marking the second locations with a second visual effect, which is visually distinct from the first visual effect, at positions on the image that are determined in accordance with the second registration.

There is also provided a method, including performing a first registration of a tracking system, which is configured to track a location of a probe within an organ of a human body, with a baseline coordinate system, measuring first locations of the probe within the organ following the first registration, presenting, on an image of the organ, first indicators marking the first locations with a first visual effect, at positions on the image that are determined in accordance with the first registration, after measuring the first locations, performing a second registration of the tracking system with the baseline coordinate system, measuring second locations of the probe within the organ following the second registration, and presenting, on the image of the organ, second indicators marking the second locations with a second visual effect, which is visually distinct from the first visual effect, at positions on the image that are determined in accordance with the second registration.

In embodiments of the present invention, the tracking system and the baseline system are each selected from a group consisting of a field-based location tracking system, an impedance-based location tracking system and a medical imaging system. In some embodiments, the image includes a simulated surface of the organ. In additional embodiments, the first visual effect includes a first color and the second visual effect includes a second color different from the first color.

In further embodiments, performing the first registration includes identifying a relationship between the tracking system and the baseline coordinate system, and the second registration is performed upon detecting a change in the relationship. In supplementary embodiments, the probe includes a catheter, and the organ includes a heart. In some embodiments, the locations include ablation locations.

There is further provided, in accordance with an embodiment of the present invention, a computer software product for sensing, using a baseline coordinate system and a tracking system configured to track a location of a probe within an organ of a human body, the product including a non-transitory computer-readable medium, in which program instructions are stored, which instructions, when read by a computer, cause the computer to perform a first registration of the tracking system, with the baseline coordinate system, to measure first locations of the probe within the organ following the first registration, to present, on an image of the organ, first indicators marking the first locations with a first visual effect, at positions on the image that are determined in accordance with the first registration, after measuring the first locations, to perform a second registration of the tracking system with the baseline coordinate system, to measure second locations of the probe within the organ following the second registration, and to
present, on the image of the organ, second indicators marking the second locations with a second visual effect, which is visually distinct from the first visual effect, at positions on the image that are determined in accordance with the second registration.

### BRIEF DESCRIPTION OF THE DRAWINGS

The disclosure is herein described, by way of example only, with reference to the accompanying drawings, wherein:
Figure 1 is a schematic pictorial illustration of a medical system comprising multiple systems configured to track a location of a catheter in a heart while performing an ablation procedure, in accordance with an embodiment of the present invention;
Figure 2 is a schematic pictorial illustration of the catheter in the heart, in accordance with an embodiment of the present invention;
Figure 3 is a flow diagram that illustrates a method of presenting ablation locations in the heart; and
Figure 4 is a schematic pictorial illustration of the ablation locations presented on an electroanatomical map, in accordance with an embodiment of the present invention.

### DETAILED DESCRIPTION OF EMBODIMENTS

### OVERVIEW

Various diagnostic and therapeutic procedures involve mapping of the electrical potential on the inner surface of a cardiac chamber. Electrical mapping can be performed, for example, by inserting a medical probe (e.g., a catheter), whose distal end is fitted with a position sensor and a mapping electrode (also referred to herein as a probe electrode), into the cardiac chamber. The cardiac chamber is mapped by positioning the probe at multiple points on the inner chamber surface. At each point, the electrical potential is measured using the mapping electrode, and the distal end position is measured using the position sensor. The measurements are typically presented as a map of the electrical potential distribution over the cardiac chamber surface.

While positioning the medical probe within the cardiac chamber, impedance-based and/or magnetic field-based (also referred to herein as field-based) position sensing systems can be used to determine a location of the probe within the cardiac chamber. In impedance-based location sensing systems, such as those described in U.S. Patent 8,456,182 a set of adhesive skin patches is affixed to a subject's body, and a distal end of a medical probe (e.g., a catheter) is inserted into a body cavity of the subject.

The patches include respective electrodes in contact with a surface of the subject. Typically the set of patches comprises three or more patches. A control console delivers a current to an electrode (also referred to herein as an impedance-based location sensor) positioned at the distal end of the probe.

Each of the patches receives a portion of the current, and conveys its respective received current back to the control console. From the received currents the control console can determine a respective impedance between each of the patches and the mapping electrode, and compute, based on the impedances, impedance-based location coordinates for the distal end. The impedance-based location coordinates are three-dimensional coordinates measured with respect to a frame of reference defined by the patches, herein assumed to have impedance-based coordinates, and enable the distal end to be tracked in this frame of reference in the body cavity.

In field-based position sensing systems, multiple magnetic field generators may be positioned in the vicinity of the subject. A field-based position sensor, also herein termed a magnetic tracking sensor, is positioned at the distal end of the probe, and the sensor conveys a probe signal to the control console in response to the magnetic fields received from the field generators. Upon receiving the probe signal from the tracking sensor, the control console can compute, based on the probe signal, field-based probe location coordinates for the distal end. The field-based probe location coordinates are three-dimensional coordinates with respect to a frame of reference defined by field-based location coordinates of the magnetic field generators, and also enable the distal end to be tracked in the field-based frame of reference.

Field-based position sensing systems are typically more accurate than impedance-based location sensing systems. For example, field-based position sensing systems may be accurate to within one millimeter while impedance-based position systems may be accurate to within three millimeters. However, field-based systems are typically more costly than the impedance-based systems.

Medical systems, typically those using multiple probes during a medical procedure, may incorporate into at least one of the probes an electrode and a field-based position sensor. Such a probe, herein termed a reference probe, may be used to map the volume of the body cavity in both systems, and a correlation between the two mappings may then be applied to other probes having only mapping electrodes, the mapping electrodes being used for tracking the probes in an impedance-based system. In order that the impedance-based location coordinates of the reference probe correspond to its field-based location coordinates, the frames of reference of the two systems are registered, so generating a relation between the two frames of reference. Using the relation, *inter alia,* typically increases the accuracy of the impedance-based system, as well as allowing the electrode-only probes to be tracked in the field-based system.

During a medical procedure such as an ablation of tissue in a heart, the heart may move due to the patient breathing even though the patient is immobile. While breathing is overall a cyclic process, the amplitude and period of the breathing typically vary during a procedure. Such motions, including motions of the "complete" patient, may be allowed for, for example by positioning the reference probe in a fixed position within the heart, and tracking location changes of the heart with this probe.

Upon detecting motion of the patient during the procedure, the multiple coordinate systems may need to be re-registered. However, each re-registration may introduce errors into locations that were measured prior to the re-registration. In instances where there are multiple re-registrations, errors in values of earlier measured positions that were registered with earlier registrations may be more significant than errors of recently measured positions that were registered with more recent registrations.

Embodiments of the present invention provide systems for identifying one or more locations that may be more liable to error, by presenting each of the locations using a respective visual effect that is associated with its respective registration. In embodiments of the present invention, the locations are measured by a control console comprising a tracking system that is configured to track a location of a probe within an organ of a human body.

As explained hereinbelow, a first registration is performed between the tracking system and a baseline coordinate system. Examples of tracking and baseline coordinate systems include, but are not limited to, field-based location tracking systems, impedance-based location tracking systems and medical imaging systems.

Subsequent to the first registration, the control console measures first locations of the probe within the organ, and presents, on an image of the organ, first indicators marking the first locations with a first visual effect, at positions on the image that are determined in accordance with the first registration. After measuring the first locations, a second registration is performed between the tracking system and the baseline coordinate system, and upon measuring second locations of the probe within the organ, the control console presents, on the image of the organ, second indicators marking the second locations with a second visual effect, which is visually
distinct from the first visual effect, at positions on the image that are determined in accordance with the second registration.

In embodiments where the visual effects comprise different colors, the locations can be "color-coded" by presenting the first locations measured using a first color, and presenting the second locations using a second color. By color-coding the locations, systems implementing embodiments of the present invention enable a physician to decide whether or not to re-measure any particular location.

### SYSTEM DESCRIPTION

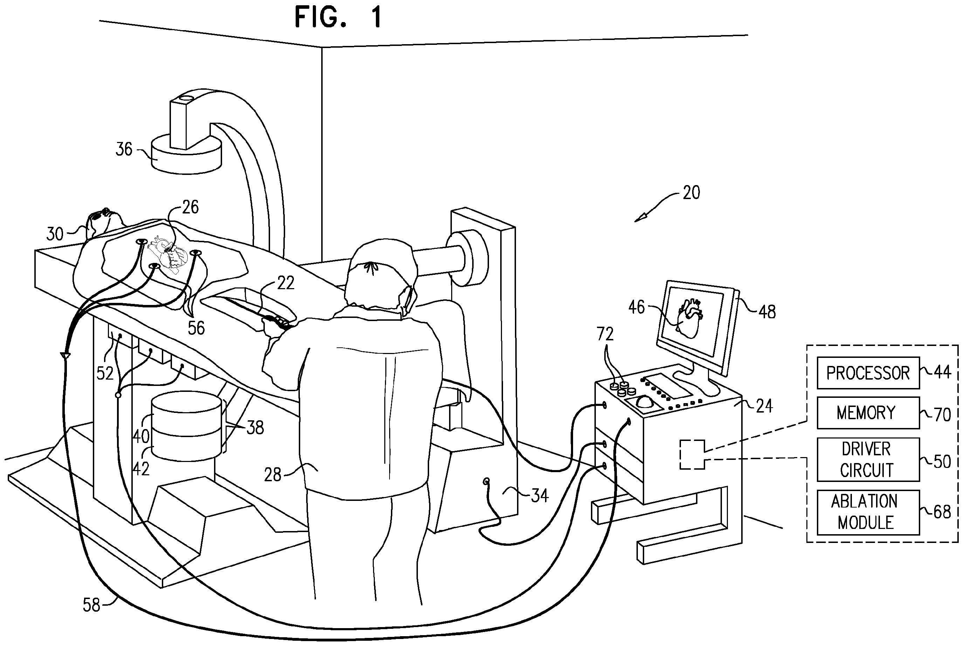

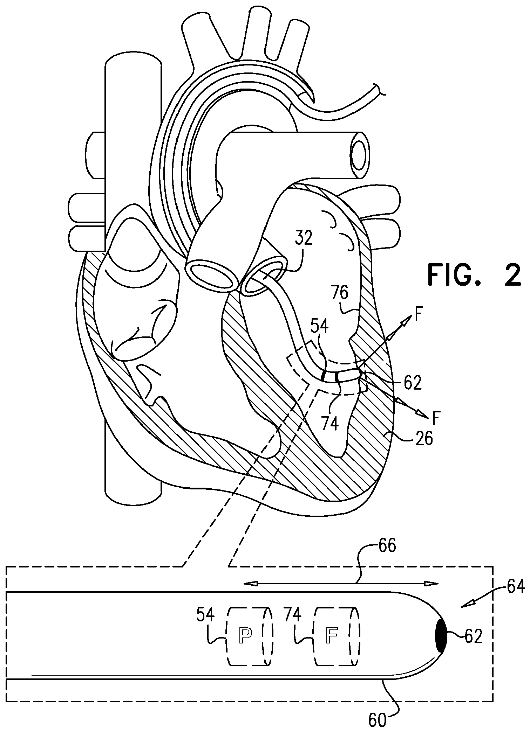

Figure 1 is a schematic pictorial illustration of a medical system 20 comprising a medical probe 22 and a control console 24, and Figure 2 is a schematic illustration of the medical probe inside a chamber of a heart 26, in accordance with an embodiment of the present invention. System 20 may be based, for example, on the CARTO® system, produced by Biosense Webster Inc. (Diamond Bar, California). In embodiments described hereinbelow, it is assumed that probe 22 is used for diagnostic or therapeutic treatment, such as performing ablation of heart tissue in heart 26. Alternatively, probe 22 may be used, *mutatis mutandis,* for other therapeutic and/or diagnostic purposes in the heart or in other body organs.

An operator 28 inserts probe 22 through the vascular system of a patient 30 so that a distal end 32 (Figure 2) of probe 22 enters a chamber of heart 26. In the configuration shown in Figure 1, operator 28 uses a fluoroscopy unit 34 to visualize distal end 32 inside heart 26. Fluoroscopy unit 34 comprises an X-ray source 36, positioned above patient 30, which transmits X-rays through the patient. A flat panel detector 38, positioned below patient 30, comprises a scintillator layer 40 which converts the X-rays which pass through patient 30 into light, and a sensor layer 42 which converts the light into electrical signals. Sensor layer 42 typically comprises a two dimensional array of photodiodes, where each photodiode generates an electrical signal in proportion to the light detected by the photodiode.

Control console 24 comprises a processor 44 that converts the signals from fluoroscopy unit 34 and probe 22 into an electroanatomical map 46 (also referred to herein as image 46), which comprises information regarding the procedure that the processor presents on a display 48. Display 48 is assumed, by way of example, to comprise a cathode ray tube (CRT) display or a flat panel display such as a liquid crystal display (LCD), light emitting diode (LED) display or a plasma display. However other display devices can also be employed to implement embodiments of the present invention. In some embodiments, display 48 may comprise a touchscreen configured to accept inputs from operator 28, in addition to presenting electroanatomical map 46.

System 20 can use field-based position sensing to determine position coordinates of distal end 32 inside heart 26. In configurations where system 20 uses field-based based position sensing, console 24 comprises a driver circuit 50 which drives field generators 52 to generate magnetic fields within the body of patient 30. Typically, field generators 52 comprise coils, which are placed below the patient at known positions external to patient 30. These coils generate magnetic fields in a predefined working volume that contains heart 26. A magnetic field sensor 54 (also referred to herein as position sensor 54) within distal end 32 of probe 22 generates electrical signals in response to the magnetic fields from the coils, thereby enabling processor 44 to determine the position of distal end 32 within the cardiac chamber. Magnetic field-based position tracking techniques are described, for example, in U.S. Patents 5,391,199, 6,690,963, 5,443,489, 6,788,967, 5,558,091, 6,172,499 and 6,177,792.

In an alternative embodiment, the roles of location sensor 54 and magnetic field generators 52 may be reversed. In other words, driver circuit 50 may drive a magnetic field generator in distal end 32 to generate one or more magnetic fields. The coils in ach field generator 52 may be configured to sense the fields and generate signals indicative of the amplitudes of the components of these magnetic fields. Processor 44 can receive and process these signals in order to determine the position coordinates of distal end 32 within heart 26.

During a medical procedure, an array of adhesive skin patches 56 are affixed to patient 30. In other words, each of the skin patches in the array is affixed to a surface of the body of patient 30. At least one of the patches comprises one or more magnetic field sensors (e.g., coils) that can measure the magnetic fields produced by field generators 52, and responsively convey the magnetic field measurements to console 24. Based on the magnetic measurements received from the magnetic field sensors (also referred to herein as patch sensors) in a given patch 56, processor 44 can determine a current field-based position, relative to the field generators, of the given skin patch.

Each patch 56 also comprises a body surface electrode in contact with the surface of the body, and console 24 is connected by a cable 58 to the body surface electrodes. In the configuration shown in Figure 2, distal end 32 is enveloped by an insulating exterior surface 60, and comprises a probe electrode 62 that typically comprises one or more thin metal layers formed over the insulating exterior surface at a distal tip 64 of probe 22.

In operation, processor 44 can determine impedance-based location coordinates of distal end 32 inside heart 26 based on the impedances measured between patches 56 and probe electrode 62. Impedance-based position tracking techniques are described, for example, in U.S. Patents 5,983,126, 6,456,864 and 5,944,022. In embodiments of the present invention, processor 44 can use a distance 66 between position sensor 54 and electrode 62 when performing a registration between the position-dependent magnetic signals received from the magnetic field sensor and position-dependent electrical signals (i.e., impedance measurements) received from patches 56.

In some embodiments, system 20 can use electrode 62 for both impedance-based location sensing and for other activities such as potential acquisition for electrical mapping of the heart, or ablation. Console 24 also comprises a radio frequency (RF) ablation module 68 that delivers electrical power to electrode 62. Processor 44 uses ablation module 68 to monitor and control ablation parameters such as the level of ablation power applied via electrode 62. Ablation module 68 may also monitor and control the duration of the ablation that is provided.

Processor 44 receives and processes the signals generated by position sensor 54 in order to determine field-based position coordinates of distal end 32, typically including both field-based location and field-based orientation coordinates. In addition to determining the field-based coordinates of distal end 32, processor 44 can also receive and process impedances from patches 56 in order to determine impedance-based location coordinates of the distal end. The method of position sensing described hereinabove is implemented in the above-mentioned CARTO® system and is described in detail in the patents and patent application cited above.

Based on the signals received from probe 22 and other components of system 20, processor 44 drives display 48 to update map 46, so as to present a current position of distal end 32 in the patient's body, as well as to present status information and guidance regarding the procedure that is in progress. Processor 44 stores data representing map 46 in a memory 70. In some embodiments, operator 28 can manipulate map 46 using one or more input devices 72. In embodiments, where display 48 comprises a touchscreen display, operator 28 can manipulate map 46 via the touchscreen display.

In the configuration shown in Figure 2, probe 22 also comprises a force sensor 74 contained within distal end 32. Force sensor 74 measures a force F applied by distal tip 64 on endocardial tissue 76 of heart 26 by generating a signal to the console that is indicative of the force exerted by the distal tip on the endocardial tissue. While performing an ablation, processor 44 can measure force F to verify contact between distal tip 64 and endocardial tissue 76.

In one embodiment, force sensor 74 may comprise a magnetic field transmitter and receiver connected by a spring in distal tip 64, and may generate an indication of the force based on measuring the deflection of the spring. Further details of this sort of probe and force sensor are described in U.S. Patent Application Publications 2009/0093806 and 2009/0138007. Alternatively, distal end 32 may comprise another type of force sensor.

Processor 44 typically comprises a general-purpose computer, with suitable front end and interface circuits for receiving signals from probe 22 and controlling the other components of console 24. Processor 44 may be programmed in software to carry out the functions that are described herein. The software may be downloaded to console 24 in electronic form, over a network, for example, or it may be provided on non-transitory tangible media, such as optical, magnetic or electronic memory media. Alternatively, some or all of the functions of processor 44 may be carried out by dedicated or programmable digital hardware components.

### PRESENTING RE-REGISTERED MAP POINTS

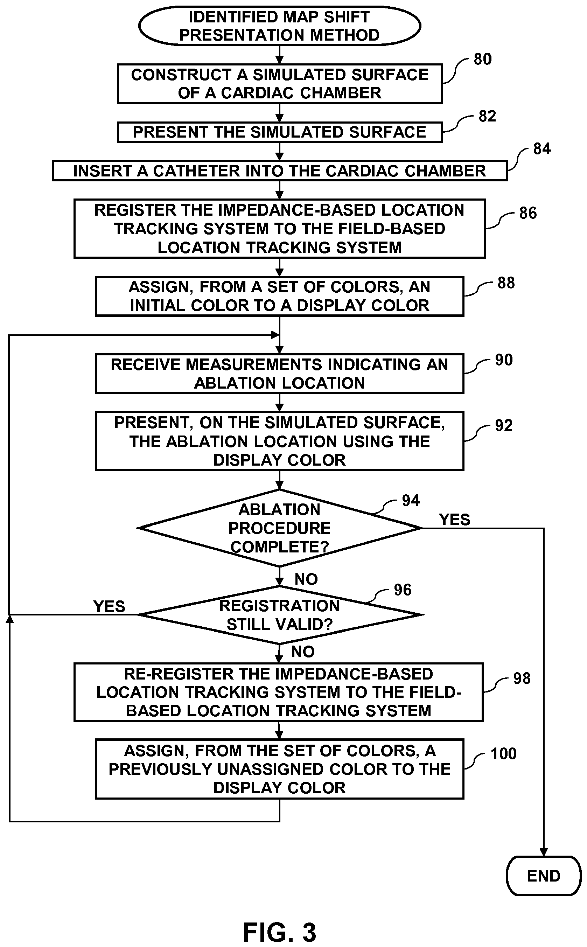

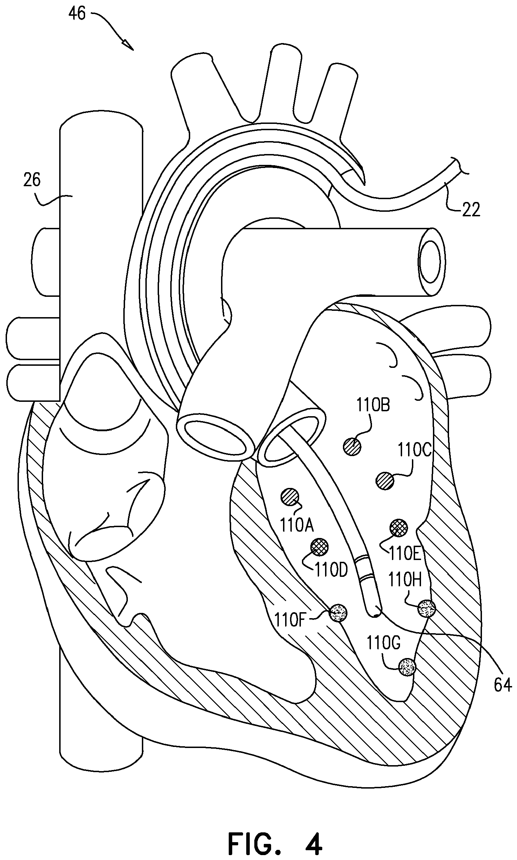

The following description assumes, by way of example, that an ablation procedure is performed on patient 30. Those having ordinary skill in the art will be able to adapt the description, *mutatis mutandis,* for other procedures such as a cardiac chamber mapping procedure. In the ablation procedure, processor 44 can generate electroanatomical map 46 comprising map points comprising ablation locations collected from probe 22. Each map point may comprise a respective coordinate within a body cavity, and possibly a physiological property collected by probe 22 at the respective coordinate. While the description referencing Figures 3 and 4 hereinbelow assumes map points 110 represent ablation locations in heart 26, map points representing any other types of locations mapped by probe 22, such as a location on a surface, in any organ of patient 30 are considered to be within the scope of the present invention.

Figure 3 is a flow diagram that illustrates a method of identifying and presenting shifts in map 46, and Figure 4 is a schematic pictorial illustration of the electroanatomical map comprising map points 110 (also referred to herein as indicators), in accordance with an embodiment of the present invention. In Figure 4, map points 110 are differentiated by appending a letter to the identifying numeral, so that the map points comprise map points 110A-110G.

In a construction step 80, processor 44 constructs a simulated surface of the cardiac chamber. In some embodiments, processor 44 constructs the simulated surface based on additional map points (not shown), typically three-dimensional mapping points of the surface, previously collected from probe 22. In a presentation step 82, the processor presents, on display 48, electroanatomical map 46 comprising the simulated surface.

In an insertion step 84, operator 28 inserts distal end 32 of probe 22 into the cardiac chamber. As operator 28 maneuvers probe 22, processor 44 determines current impedance based location coordinates for distal end 32 based on an impedance between patches 56 and electrode 62, and determines field-based location coordinates for the distal end 32 based on signals received from position sensor 54. In an initial registration step 86, processor identifies a relationship between the impedance-based location coordinates and the field-based location coordinates, and uses the relationship to register the impedance-based location coordinates to the field-based location coordinates. A method for determining a relationship such as that referred to above is provided in U.S. Patent 8,456,182, to Bar-Tal et al..

During the medical procedure, as processor 44 collects map points 110, the processor may detect a change in the current relationship between the impedance-based location coordinates and the field-based location coordinates of distal end 32. Such a change may be caused, for example, by movement of patient 30. Upon detecting the change in the relationship, processor 44 can re-register the impedance-based location coordinates to the field-based location coordinates. In embodiments of the present invention, upon re-registering the impedance-based location coordinates to the field-based location coordinates, processor 44 changes the color used to present ablation locations collected subsequent to the re-registration. Therefore, when initializing system 20, processor 44 can define a set of colors that can be used to present the ablation locations on display 48.

In a first assignment step 88, processor 44 assigns, from the set of colors, an initial color to a display color. As operator 28 uses probe 22 to perform an ablation on intracardiac tissue 76, in a receive step 90, processor 44 receives, from probe 22, measurements indicating a given ablation location marked by a given map point 110. In some embodiments, the measurements comprise impedances between patches 56 and electrode 62, and processor 44 can use the registration to determine or measure (i.e., find coordinates for) the given map point.

In a presentation step 92, processor 44 presents, on display 48, electroanatomical map 46 comprising a fusion of the simulated surface and the given ablation location. In embodiments of the present invention, processor 44 calculates the given ablation location using the (current) registration, and presents the given ablation location in heart 26 using the assigned display color. In a first comparison step 94, if the ablation procedure is not complete, then in a second comparison step 96, processor 44 checks to see if the current registration is still valid.

If processor 44 detects a change in the relationship between the impedance-based location coordinates and the field-based location coordinates, then the current registration is not valid, and in a re-registration step 98, the processor uses the changed relationship to re-register the impedance-based location coordinates to the field-based location coordinates. In a second assignment step 100, processor 44 processor 44 assigns, from the set of colors, a previously unassigned color to the display color, and the method continues with step 92.

Returning to step 96, if the current registration is still valid then the method continues with step 90. Returning to step 94, if the ablation procedure is complete, then the method ends.

In embodiments of the present invention, as described in the description referencing Figure 3 hereinabove, processor 44 assigns a unique visual effect (e.g., a given color from the set of colors) to the ablation locations collected with each given registration. In the example shown in Figure 4, processor 44 collects ablation locations 110A-110C using a first registration and presents locations 110A-110C in map 46 using a first visual effect, collects ablation locations 110D and 110E using a second registration and presents locations 110D and 110E in the electroanatomical map using a second visual effect, and collects ablation locations 110F-110H using a third registration and presents locations 110F-110H in the electroanatomical map using a third visual effect. In the example shown in Figure 4, the visual effects comprise "fill" (also known as "hatch") patterns. In additional embodiments, processor 44 can use other types of visual effects such as color, intensity, size of indicators 110, and blinking attributes to differentiate between ablation locations measured with different registrations.

The example in the description referencing Figure 3 hereinabove describes presenting ablation locations received from a tracking system comprising an impedance-based location tracking system that is registered to a baseline coordinate system comprising a field-based location tracking system. Presenting map points collected using other types of tracking systems that are registered to other types of baseline coordinate systems is considered to be within the scope of the present invention. For example, each of the tracking and baseline coordinate systems may comprise an impedance-based tracking system, a field-based tracking system, or a medical imaging system such as fluoroscopy unit 34. Therefore in the configuration shown in Figures 1 and 2, the impedance-base location coordinates may be registered to field-based location coordinates (or vice-versa), the impedance-based location coordinates may be registered to image-based location coordinates in electroanatomical map 46 (or vice-versa) that processor 44 generates using image data received from fluoroscopy unit 34, or the field-based location coordinates can be registered to the image-based location coordinates (or vice-versa). Examples of additional medical imaging systems that may be configured as tracking systems and/or baseline coordinate systems include, but are not limited to ultrasonic imaging systems or a magnetic resonance imaging (MRI) systems and computed tomography (CT) imaging systems.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. An apparatus, comprising:
a tracking system configured to track a location of a probe (22) within an organ of a human body;
a baseline coordinate system;
a display (48); and
a processor (44) configured:
to perform a first registration of the tracking system with the baseline coordinate system,
to measure first locations of the probe within the organ following the first registration,
to present, on the display, first indicators (110A-110C) on an image of the organ marking the first locations with a first visual effect, at positions on the image that are determined in accordance with the first registration,
after measuring the first locations, to perform a second registration of the tracking system with the baseline coordinate system,
to measure second locations of the probe within the organ following the second registration, and
**characterised in that** the processor is further configured to present, on the image of the organ, second indicators (110D, 110E) marking the second locations with a second visual effect, which is visually distinct from the first visual effect, at positions on the image that are determined in accordance with the second registration.

2. The apparatus according to claim 1, wherein the tracking system and the baseline system are each selected from a group consisting of a field-based location tracking system, an impedance-based location tracking system and a medical imaging system.

3. The apparatus according to claim 1, wherein the image comprises a simulated surface of the organ.

4. The apparatus according to claim 1, wherein the first visual effect comprises a first color and the second visual effect comprises a second color different from the first color.

5. The apparatus according to claim 1, wherein the processor (44) is configured to perform the first registration by identifying a relationship between the tracking system and the baseline coordinate system, and wherein the processor (44) is configured to perform the second registration upon detecting a change in the relationship.

6. The apparatus according to claim 1, wherein the probe (22) comprises a catheter, and the organ comprises a heart.

7. The apparatus according to claim 1, wherein the locations comprise ablation locations.

8. A computer software product for sensing, using a baseline coordinate system and a tracking system configured to track a location of a probe (22) within an organ of a human body, the product comprising a non-transitory computer-readable medium, in which program instructions are stored, which instructions, when read by a computer, cause the computer:
to perform a first registration of the tracking system, with the baseline coordinate system;
to measure first locations of the probe within the organ following the first registration;
to present, on an image of the organ, first indicators (110A-110C) marking the first locations with a first visual effect, at positions on the image that are determined in accordance with the first registration;
after measuring the first locations, to perform a second registration of the tracking system with the baseline coordinate system;
to measure second locations of the probe within the organ following the second registration; and
to present, on the image of the organ, second indicators (110D, 110E) marking the second locations with a second visual effect, which is visually distinct from the first visual effect, at positions on the image that are determined in accordance with the second registration.

## Patentansprüche

1. Gerät, das Folgendes umfasst:
ein Tracking-System, das zur Nachverfolgung eines Aufenthaltsortes einer Sonde (22) innerhalb eines Organs eines menschlichen Körpers konfiguriert ist;
ein Basiskoordinatensystem;
eine Anzeige (48); und
einen Rechner (44), der konfiguriert ist, um:
eine erste Registrierung des Tracking-Systems bei dem Basiskoordinatensystem durchzuführen,
die ersten Aufenthaltsorte der Sonde innerhalb des Organs nach der ersten Registrierung zu messen,
erste Indikatoren (110A-110C) auf einem Bild des Organs auf der Anzeige darzustellen, wobei die ersten Aufenthaltsorte mit einem ersten optischen Effekt markiert werden, an Positionen auf dem Bild, der gemäß der ersten Registrierung bestimmt werden,
nach dem Messen der ersten Aufenthaltsorte, eine zweite Registrierung des Trackingsystems mit dem Basiskoordinatensystem durchzuführen,
nach der zweiten Registrierung, zweite Aufenthaltsorte der Sonde innerhalb des Organs zu messen, und
**dadurch gekennzeichnet, dass** der Rechner ferner konfiguriert ist, um auf dem Bild des Organs, zweite Indikatoren (110D, 110E) anzuzeigen, welche die zweiten Aufenthaltsorte mit einem zweiten optischen Effekt markieren, der sich optisch von dem ersten optischen Effekt unterscheidet, an Positionen auf dem Bild, die gemäß der zweiten Registrierung bestimmt werden.

2. Gerät nach Anspruch 1, wobei das Tracking-System und das Basissystem jeweils ausgewählt sind aus einer Gruppe, bestehend aus einem Feld-basierten-Aufenthaltsort-Tracking-System, einem Impedanzbasierten-Aufenthaltsort-Tracking-System, und einem medizinischen Bildgebungssystem.

3. Gerät nach Anspruch 1, wobei das Bild eine simulierte Oberfläche des Organs umfasst.

4. Gerät nach Anspruch 1, wobei der erste optische Effekt eine erste Farbe umfasst und der zweite optische Effekt eine zweite Farbe umfasst, die sich von der ersten Farbe unterscheidet.

5. Gerät nach Anspruch 1, wobei der Vorläufer (44) derart konfiguriert ist, dass er die erste Registrierung durchführt, indem ein Verhältnis zwischen dem Tracking-System und dem Basiskoordinatensystem identifiziert wird, und wobei der Vorläufer (44) konfiguriert ist, um die zweite Registrierung auf die Detektion einer Veränderung des Verhältnisses durchzuführen.

6. Gerät nach Anspruch 1, wobei die Sonde (22) einen Katheter umfasst und das Organ ein Herz umfasst.

7. Gerät nach Anspruch 1, wobei die Aufenthaltsorte Ablationsorte umfassen.

8. Computersoftwareprodukt zur Erfassung, unter Verwendung eines Basiskoordinatensystems und eines Trackingsystems, das konfiguriert ist, um einen Aufenthaltsort einer Sonde (22) innerhalb eines Organs eines menschlichen Körpers nachzuverfolgen, wobei das Produkt ein nicht-flüchtiges computerlesbares Medium umfasst, in welchem die Programmanweisungen gespeichert sind, wobei die Anweisungen, wenn sie durch einen Computer gelesen werden, den Computer zu Folgendem veranlassen:
Durchführen einer ersten Registrierung des Trackingsystems, an dem Basiskoordinatensystem;
Messen erster Aufenthaltsorte der Sonde innerhalb des Organs nach der ersten Registrierung;
Darstellen, auf einem Bild des Organs, erster Indikatoren (110A-110C), welche erste Aufenthaltsorte mit einem ersten optischen Effekt markieren, an Positionen auf dem Bild, die gemäß der ersten Registrierung bestimmt werden;
nach dem Messen der ersten Aufenthaltsorte, Durchführen einer zweiten Registrierung des Tracking-Systems an dem Basiskoordinatensystem;
Messen zweiter Aufenthaltsorte der Sonde innerhalb des Organs nach der zweiten Registrierung; und
Darstellen, auf einem Bild des Organs, zweiter Indikatoren (110D, 110E), welche zweite Aufenthaltsorte mit einem zweiten optischen Effekt markieren, der sich optisch von dem ersten optischen Effekt unterschiedet, an Positionen auf dem Bild, die gemäß der zweiten Registrierung bestimmt werden.

## Revendications

1. Appareil, comprenant :
un système de suivi configuré pour suivre un emplacement d'une sonde (22) dans un organe d'un corps humain ;
un système de coordonnées de base ;
un affichage (48) ; et
un processeur (44) configuré :
pour effectuer un premier enregistrement du système de suivi avec le système de coordonnées de base, afin de mesurer les premiers emplacements de la sonde dans l'organe après le premier enregistrement,
pour présenter, sur l'affichage, les premiers indicateurs (110A-110C) sur une image de l'organe marquant les premiers emplacements avec un premier effet visuel, aux emplacements sur l'image qui sont déterminés en fonction du premier enregistrement,
après avoir mesuré les premiers emplacements, pour effectuer un deuxième enregistrement du système de suivi avec le système de coordonnées de base,
pour mesurer les deuxièmes emplacements de la sonde dans l'organe après le deuxième enregistrement, et
**caractérisé en ce que** le processeur est en outre configuré
pour présenter, sur l'image de l'organe, des deuxièmes indicateurs (110D, 110E) marquant les deuxièmes emplacements avec un deuxième effet visuel, visuellement distinct du premier effet visuel, aux emplacements sur l'image qui sont déterminés en fonction du deuxième enregistrement.

2. Appareil selon la revendication 1, le système de suivi et le système de base étant chacun choisis dans un groupe constitué d'un système de suivi de localisation basé sur le champ, d'un système de suivi de localisation basé sur l'impédance et d'un système d'imagerie médicale.

3. Appareil selon la revendication 1, l'image comprenant une surface simulée de l'organe.

4. Appareil selon la revendication 1, le premier effet visuel comprenant une première couleur et le deuxième effet visuel comprenant une deuxième couleur différente de la première couleur.

5. Appareil selon la revendication 1, le processeur (44) étant configuré pour effectuer le premier enregistrement en identifiant une relation entre le système de suivi et le système de coordonnées de base, et le processeur (44) étant configuré pour effectuer le deuxième enregistrement en détectant un changement dans la relation.

6. Appareil selon la revendication 1, la sonde (22) comprenant un cathéter et l'organe comprenant un coeur.

7. Appareil selon la revendication 1, les emplacements comprenant des emplacements d'ablation.

8. Produit logiciel informatique pour détecter, en utilisant un système de coordonnées de base et un système de suivi configuré pour suivre un emplacement d'une sonde (22) dans un organe d'un corps humain, le produit comprenant un support non transitoire lisible par ordinateur, dans lequel des instructions de programme sont stockées, lesquelles instructions, quand elles sont lues par un ordinateur, amènent l'ordinateur à :
effectuer un premier enregistrement du système de suivi, avec le système de coordonnées de base ;
mesurer les premiers emplacements de la sonde dans l'organe après le premier enregistrement ;
présenter, sur une image de l'organe, les premiers indicateurs (110A-110C) marquant les premiers emplacements avec un premier effet visuel, aux emplacements sur l'image qui sont déterminés en fonction du premier enregistrement ;
après avoir mesuré les premiers emplacements, effectuer un deuxième enregistrement du système de suivi avec le système de coordonnées de base ;
mesurer les deuxièmes emplacements de la sonde dans l'organe après le deuxième enregistrement ; et
présenter, sur l'image de l'organe, des deuxièmes indicateurs (110D, 110E) marquant les deuxièmes emplacements avec un deuxième effet visuel, visuellement distinct du premier effet visuel, aux emplacements sur l'image qui sont déterminés en fonction du deuxième enregistrement.
